# FASCICULE DE BREVET EUROPEEN

(11) **EP 1 398 009 B1**
(45) Date de publication et mention de la délivrance du brevet: **29.11.2006**
(21) Numéro de dépôt: 03027583.8
(22) Date de dépôt: 03.08.2000
(51) Int. Cl.: A61F 2/42, A61B 17/17, A61F 2/46

(54) **Matériel ancillaire de pose d'un implant malléolaire pour prothèse partielle ou totale de cheville et implant apte à être posé avec ce matériel**
Hilfsinstrument zum Einsetzen eines malleolären Implantats für eine Teil- oder Totalknöchelprothese und Implantat welches geeignet ist, mit diesem Instrument eingesetzt zu werden
Ancillary equipment for inserting a malleolar implant for a partial or total ankle prosthesis and implant suitable for being inserted with this equipment

(30) Priorité: 05.08.1999 FR 9910340
(43) Date de publication de la demande: 17.03.2004
(62) Demande divisionnaire de: 00420173.7
(73) Titulaire: TORNIER, 38330 Saint-Ismier (FR)
(72) Inventeur: Tornier, Alain, 38330 Saint Ismier (FR); Bonnin, Michel, 69006 Lyon (FR); Colombier, Jean-Alain, 31130 Balma (FR); Judet, Thierry, 92410 Ville d'Avray (FR)
(74) Mandataire: Myon, Gérard Jean-Pierre

(56) Documents cités:
- FR-A- 2 700 462
- US-A- 4 159 716
- US-A- 4 235 428
- US-A- 5 601 550

## Description

L'invention a trait à un matériel ancillaire de pose d'un implant malléolaire pour une prothèse partielle ou totale de la cheville ainsi qu'à un implant pouvant être posé à l'aide de ce matériel.

Les matériels ancillaires connus, par exemple de US-A-4,159,716, US-A-4,235,428 ou FR-A-2 700 462, ne permettent pas de positionner précisément un implant malléolaire par rapport à la malléole péronière.

L'invention concerne un matériel ancillaire de pose d'un implant malléolaire qui comprend un bloc-entretoise, apte à être inséré entre le tibia et l'astragale d'une cheville, et une patte solidaire de ce bloc-entretoise et s'étendant jusqu'au voisinage de la surface externe de la malléole péronière lorsque le bloc-entretoise est en place entre le tibia et l'astragale, cette patte supportant un guide de perçage de la malléole à partir de sa surface externe.

Grâce à l'invention, le perçage de la malléole péronière peut être effectué à partir de sa surface externe et en direction de sa surface interne, avec un positionnement relatif déterminé par rapport au tibia et à l'astragale, de telle sorte que la position de l'implant malléolaire en place dans ce perçage est déterminée avec précision par rapport aux surfaces articulaires respectives de l'astragale ou du tibia ou de composants prothétiques correspondants. Le bloc-entretoise peut être prévu pour coopérer avec des surfaces articulaires naturelles du tibia et/ou de l'astragale ou avec des surfaces créées par résection de ces os, dans le cas de la pose d'une prothèse totale de cheville.

Selon un aspect avantageux de l'invention, le bloc-entretoise est pourvu d'un logement de réception d'une cale d'épaisseur adaptée à l'écart entre les surfaces inférieure du tibia et supérieure de l'astragale. Cet aspect de l'invention permet de maintenir un écart, correspondant à celui qui sera ultérieurement crée par des éléments prothétiques montés en partie basse du tibia et en partie supérieure de l'astragale, lors de la détermination de la position de l'implant malléolaire.

Selon un autre aspect avantageux de l'invention, la patte est articulée sur le bloc-entretoise, avec une possibilité de pivotement limitée. Ceci permet d'ajuster la position du guide de perçage de la malléole autour de l'axe de pivotement de la patte par rapport au bloc-entretoise. Dans ce cas, le bloc-entretoise et la patte sont avantageusement pourvus d'orifices de passage d'un axe de pivotement commun.

On peut en outre prévoir que le guide de perçage est associé à un dispositif de serrage de la malléole contre une surface d'appui formée sur la patte ou le bloc-entretoise. Ceci permet une immobilisation ferme de la malléole péronière lors de son perçage et assure ainsi la précision de l'opération de perçage.

L'invention concerne également un implant malléolaire qui peut être posé à l'aide d'un matériel ancillaire tel que mentionné ci-dessus, cet implant étant défini à la revendication 10 ci-annexée.

L'invention sera mieux comprise et d'autres avantages de celle-ci apparaîtront plus clairement à la lumière de la description qui va suivre d'un mode de réalisation d'un implant malléolaire et de son matériel ancillaire de pose conformes à l'invention, donnée uniquement à titre d'exemple et faite en référence aux dessins annexés dans lesquels :
- la figure 1 est une vue en perspective d'un implant conforme à l'invention ;
- la figure 2 est une vue en perspective de l'implant de la figure 1 en cours de mise en place dans une malléole péronière représentée avec arrachement ;
- la figure 3 est une représentation schématique de principe, avec arrachements partiels, d'un matériel ancillaire de pose de l'implant de la figure 1 en cours d'utilisation et
- la figure 4 est une vue en perspective par en dessous du matériel de la figure 1.

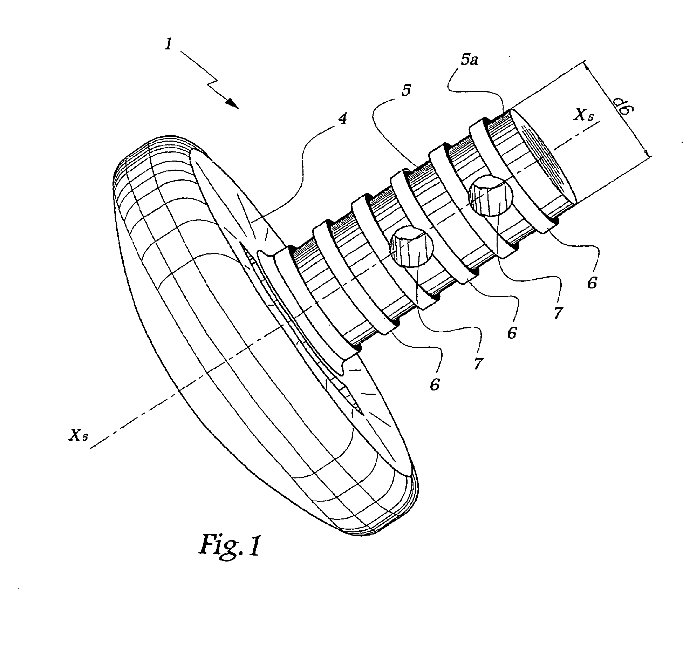

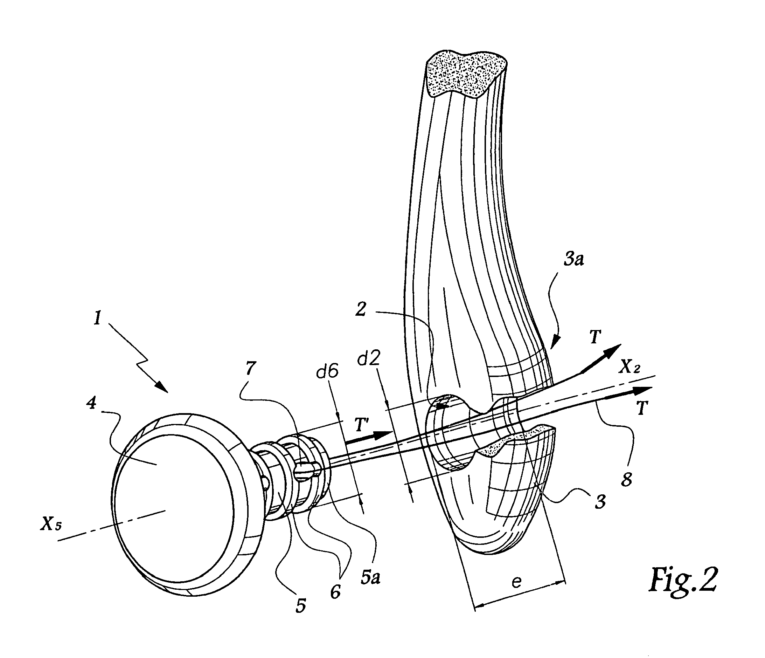

L'implant 1 représenté aux figures 1 et 2 est destiné à être introduit dans un perçage 2 ménagé dans la malléole externe ou péronière 3. L'implant 1 comprend une tête bombée 4, sensiblement selon une forme de calotte sphérique et dont le rayon de courbure est globalement égal à celui de la joue externe de l'astragale de la cheville considérée. La queue 5 de l'implant 1 est pourvue de collerettes radiales externes 6 dont le diamètre extérieur d₆ est sensiblement égale au diamètre interne d₂ du perçage 2.

Deux orifices 7 sont prévus dans la queue 5 et sont susceptibles de recevoir un fil de suture 8 ou un autre lien flexible. Lorsqu'un tel fil est engagé dans l'un des orifices 7, il est possible d'exercer sur le fil 8, un effort de traction T qui est transmis par le fil 8 à la queue 5 comme représenté par la flèche T' à la figure 2. Ainsi, en tirant sur le fil 8, le chirurgien introduit la queue 5 dans le perçage 2 sans devoir exercer un effort de poussée sur la tête 4 qui peut être difficilement accessible du fait du système ligamentaire environnant.

En d'autres termes, il suffit au chirurgien de placer un fil dans un des orifices 7, de passer les deux brins du fil 8 dans le perçage 2 par la face interne de la malléole, puis de tirer les brins par le côté externe de la malléole. La traction sur le fil 8 a pour effet d'introduire la queue 5 de l'implant 1 dans le perçage 2 et de plaquer la tête 4 sur l'os. L'effort de traction T exercé sur le fil 8 peut être intense et dirigé parallèlement à l'axe longitudinale X₂ du perçage 2, de telle sorte que la queue est efficacement tirée vers l'intérieur du perçage 2. En particulier, compte tenu de la direction et de l'intensité de l'effort de traction T, on peut prévoir que les diamètres d₂ et d₆ sont sensiblement égaux, de telle sorte que la queue 5 est fermement maintenue en position après sa mise en place.

La queue 5 est pourvue de deux perçages 7 répartis selon son axe X₅, le perçage 7 le plus proche de l'extrémité 5a de la queue 5 étant utilisé. Le fait que la queue 5 comporte plusieurs orifices 7 permet d'utiliser un orifice 7 relativement proche de l'extrémité 5a de la queue 5 et de disposer d'un tel orifice y compris lorsque la queue 5 est sectionnée pour adapter sa longueur à l'épaisseur e de la malléole 3. Bien entendu, le nombre de perçages 7 peut être augmenté si nécessaire.

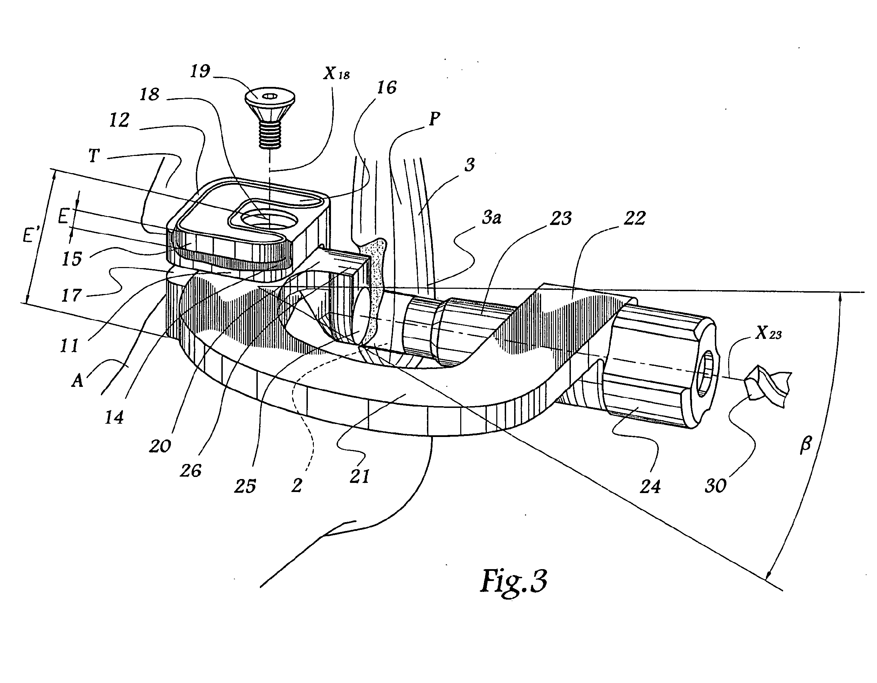

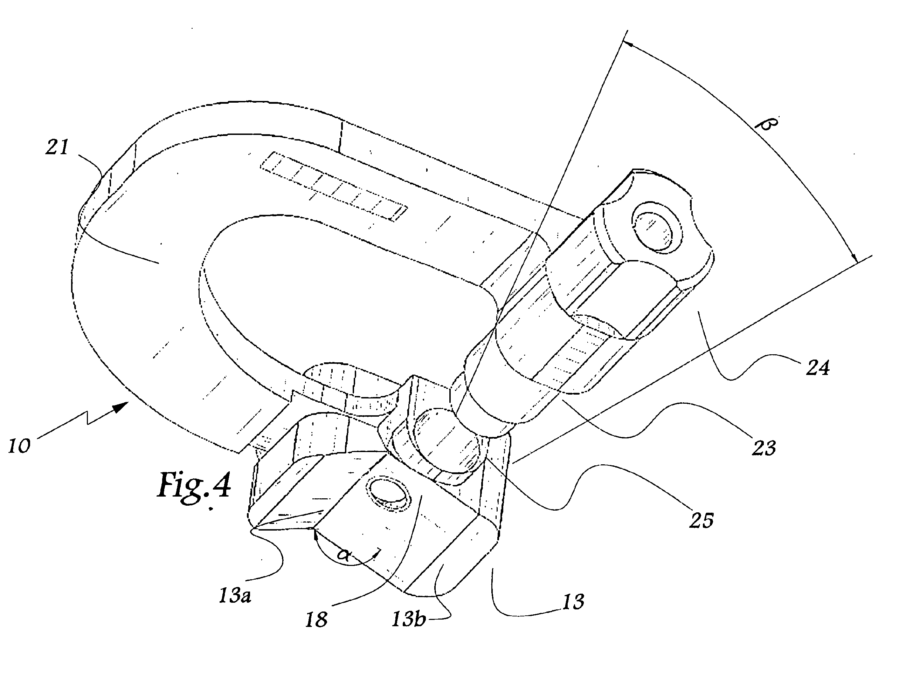

Le perçage 2 est réalisé par la face externe 3a de la malléole 3 grâce au matériel ancillaire représenté aux figures 3 et 4. Ce matériel 10 comprend un bloc-entretoise 11 prévu pour être disposé entre le tibia T et l'astragale A d'une cheville à équiper en implant 1. Le bloc 11 comprend une surface supérieure 12 sensiblement plane destinée à coopérer avec une surface plane créée par résection de la l'extrémité distale du tibia. La surface inférieure 13 du bloc 11 est formée de deux surfaces planes 13a et 13b inclinées l'une par rapport à l'autre d'un angle α, les surfaces 13a et 13b étant prévues pour venir respectivement en appui sur des surfaces correspondantes créées par résection de la patte supérieure de l'astragale A.

La surface 12 du bloc 11 comprend un logement 14 en forme de C destiné à recevoir une cale 15 dont la surface supérieure 16 est en contact avec la surface inférieure du tibia T. L'épaisseur E de la cale 15 représentée à la figure 3 est telle que sa surface supérieure 16 est affleurante avec la surface supérieure 12 du bloc 11.

Cependant, des cales d'épaisseur plus importante peuvent être utilisées lorsque l'écart E' entre les surfaces inférieure du tibia et supérieure de l'astragale est plus important que dans la configuration représentée à la figure 3.

Le bloc 11 définit un logement 17 de réception de l'extrémité 20 d'une patte 21 globalement en forme de C. L'extrémité 20 est pourvue d'un perçage non représenté qui, dans la configuration des figures 3 et 4, est aligné avec un perçage 18 ménagé dans le bloc 11 et traversant ce bloc de haut en bas, c'est-à-dire reliant les surfaces 12 et 13. Une vis 19 peut être introduite dans ce perçage qui est au moins partiellement taraudé, ce qui permet d'immobiliser l'extrémité 20 de la patte 21 à l'intérieur du logement 17. En pratique, le jeu ménagé lors du serrage de la vis 19 autorise un pivotement limité autour de l'axe X₁₈ du perçage 18.

Au niveau de son extrémité 22 opposée à l'extrémité 20, la patte 21 supporte un système de serrage 23 manoeuvrable grâce à une mollette 24 et permettant de plaquer la malléole 3 du péroné P contre une butée 25 formée sur une extension 26 de l'extrémité 20 de la patte 21. On note X₂₃ l'axe longitudinal de ces moyens de serrage. Les moyens de serrage 23 sont creux, de telle sorte qu'un forêt 30 peut être introduit jusqu'au niveau de la face externe 3a de la malléole 3 pour réaliser le perçage 2 depuis l'extérieur vers l'intérieur de la malléole 3. Ainsi, le chirurgien peut aisément viser la partie adéquate de la malléole 3 grâce aux moyens de serrage 23 qui constituent également un guide de perçage pour le forêt 30.

Comme la patte 21 est susceptible de pivoter autour de l'axe X₁₈, la position de l'axe X₂₃ est variable en pivotement autour de cet axe X₁₈, ce qui permet d'ajuster au mieux l'orientation du perçage 2 en fonction de la géométrie exacte de la malléole 3. On note *β* l'angle de pivotement maximum de l'axe X₂₃ autour de l'axe X₁₈. En pratique, l'angle *β* est de l'ordre de 10°.

Grâce au matériel 10, on peut donc former, par l'extérieur, un perçage 2 permettant une implantation rapide et efficace de l'implant 1.

Lorsque des cales 15 de hauteur supérieure à celle représentée à la figure 2 sont utilisé, il est possible de prévoir qu'elles viennent en recouvrement du perçage 18 car la vis 19 est mise en place avant le positionnement de la cale 15 qui est effectué en cours d'opération en fonction de l'écart E'.

L'invention a été représentée avec une prothèse totale de cheville, ce qui correspond à la géométrie des surfaces 12 et 13 du bloc 11. Elle est cependant applicable à une prothèse partielle de cheville, sans modification de l'implant 1, le matériel ancillaire étant alors adapté à la géométrie des surfaces anatomiques d'articulation entre le tibia et l'astragale.

## Revendications

1. Matériel ancillaire (10) de pose d'un implant malléolaire (1) **caractérisé en ce qu**'il comprend un bloc-entretoise (11), apte à être inséré entre le tibia (T) et l'astragale (A) d'une cheville, et une patte (21) solidaire dudit bloc-entretoise et s'étendant jusqu'au voisinage de la surface externe (3a) de la malléole péronière (3) lorsque le bloc-entretoise est en place entre le tibia et l'astragale, ladite patte supportant un guide de perçage (23) de la malléole à partir de sa surface externe.

2. Matériel selon la revendication 1, **caractérisé en ce que** ledit bloc-entretoise (11) est pourvu d'un logement (14) de réception d'une cale (15) d'épaisseur (E) adaptée à l'écart (E') entre les surfaces inférieure du tibia (T) et supérieure de l'astragale (A).

3. Matériel selon la revendication 2, **caractérisé en ce que** ledit logement (14) est en forme de C.

4. Matériel selon l'une des revendications 2 ou 3, **caractérisé en ce que** ladite cale (15) vient en recouvrement d'un perçage (18) de réception d'une vis (19) d'immobilisation relative dudit bloc entretoise et de ladite patte (21).

5. Matériel selon l'une des revendications 1 à 4, **caractérisé en ce que** ladite patte (21) est articulée (en 18) sur ledit bloc-entretoise (11) avec une possibilité de pivotement (β) limitée.

6. Matériel selon la revendication 5, **caractérisé en ce que** ledit bloc-entretoise (11) et ladite patte (21) sont pourvus d'orifices (18) de passage d'un axe de pivotement (19) commun.

7. Matériel selon l'une des revendications précédentes, **caractérisé en ce que** ledit guide de perçage est associé à un dispositif de serrage (23) de la malléole (3) contre une surface d'appui (25) formée sur ladite patte (21) ou ledit bloc-entretoise (11).

8. Matériel selon la revendication 7, **caractérisé en ce que** ledit dispositif de serrage (23) est creux et forme un guide de perçage pour un forêt (30) qui peut être introduit dans ledit dispositif jusqu'au niveau de la face externe (3a) de la malléole péronière (3).

9. Matériel selon l'une des revendications précédentes,
**caractérisé en ce que** la surface inférieure (13) dudit bloc-entretoise (11) est formée de deux surfaces planes (13a, 13b) inclinées (α) l'une par rapport à l'autre.

## Claims

1. Ancillary equipment (10) for inserting a malleolar implant (1), **characterised in that** it comprises a spacer block (11) adapted to be inserted between the tibia (T) and the astragalus (A) of an ankle and a lug (21) attached to said spacer block and extended as far as the vicinity of the extender surface (3a) of the peroneal malleolus (3) when the spacer block is in place between the tibia and the astragalus, said lug supporting a guide (23) for drilling into the malleolus from its external surface.

2. Equipment according to claim 1, **characterised in that** said spacer block (11) is provided with a housing (14) to receive a shim (15) of thickness (E) adapted to the distance (E') between the lower surface of the tibia (T) and the upper surface of the astragalus (A).

3. Equipment according to claim 2, **characterised in that** said housing (14) is C-shaped.

4. Equipment according to either claim 2 or claim 3, **characterised in that** said shim (15) overlaps a bore (18) for receiving a screw (19) for relative immobilisation of said spacer block and said lug (21).

5. Equipment according to any one of claims 1 to 4, **characterised in that** said lug (21) is articulated (at 18) to said spacer block (11) with limited possibility of pivoting (β).

6. Equipment according to claim 5, **characterised in that** said spacer block (11) and said lug (21) are provided with orifices (18) through which a common pivot pin (19) passes.

7. Equipment according to any one of the preceding claims, **characterised in that** said drilling guide is associated with a device (23) for clamping the malleolus (3) against a bearing surface (25) formed on said lug (21) or said spacer block (11).

8. Equipment according to claim 7, **characterised in that** said clamping device (23) is hollow and forms a drilling guide for a drill bit (30) which may be inserted into said device as far as the level of the external face (3a) of the peroneal malleolus (3).

9. Equipment according to any one of the preceding claims, **characterised in that** the lower surface (13) of said spacer block (11) is formed by two plane surfaces (13a, 13b) inclined (α) relative to each other.

## Patentansprüche

1. Hilfsinstrument (10) zum Einsetzen eines Knöchelimplantats (1), **dadurch gekennzeichnet, dass** es einen Block-Abstandhalter (11), der geeignet ist, zwischen das Schienbein (T) und das Sprungbein (A) eines Knöchels eingeführt zu werden, und eine Klammer (21) aufweist, die mit dem Block-Abstandhalter fest verbunden ist und sich bis in die Nähe der Außenfläche (3a) des Wadenbeinknöchels (3) erstreckt, wenn der Block-Abstandhalter zwischen dem Schienbein und dem Sprungbein angeordnet ist, wobei die Klammer eine Bohrungsführung (23) zum Bohren des Knöchels von seiner Außenfläche her stützt.

2. Instrument nach Anspruch 1, **dadurch gekennzeichnet, dass** der Block-Abstandhalter (11) mit einem Aufnahmesitz (14) für einen Keil (15) versehen ist, dessen Dicke (E) an den Abstand (E') zwischen der unteren Fläche des Schienbeins (T) und der oberen Fläche des Sprungbeins (A) angepasst ist.

3. Instrument nach Anspruch 2, **dadurch gekennzeichnet, dass** der Sitz (14) C-förmig ist.

4. Instrument nach Anspruch 2 oder 3, **dadurch gekennzeichnet, dass** der Keil (15) eine Bohrung (18) zur Aufnahme einer Schraube (19) zur relativen Festlegung des Block-Abstandhalters und der Klammer (21) abdeckt.

5. Instrument nach einem der Ansprüche 1 bis 4, **dadurch gekennzeichnet, dass** die Klammer (21) (bei 18) an dem Block-Abstandhalter (11) mit begrenzter Schwenkmöglichkeit (β) angelenkt ist.

6. Instrument nach Anspruch 5, **dadurch gekennzeichnet, dass** der Block-Abstandhalter (11) und die Klammer (21) mit Öffnungen (18) für den Durchgang einer gemeinsamen Schwenkachse (19) versehen sind.

7. Instrument nach einem der vorhergehenden Ansprüche, **dadurch gekennzeichnet, dass** die Bohrungsführung einer Klemmvorrichtung (23) zum Klemmen des Knöchels (3) gegen eine an der Klammer (21) oder dem Block-Abstandhalter (11) gebildete Stützfläche (25) zugeordnet ist.

8. Instrument nach Anspruch 7, **dadurch gekennzeichnet, dass** die Klemmvorrichtung (23) hohl ist und eine Bohrungsführung für einen Bohrer (30) bildet, der bis auf die Höhe der Außenfläche (3a) des Wadenbeinknöchels (3) in die Vorrichtung eingeführt werden kann.

9. Instrument nach einem der vorhergehenden Ansprüche, **dadurch gekennzeichnet, dass** die untere Fläche (13) des Block-Abstandhalters (11) aus zwei ebenen, zueinander geneigten (α) Flächen (13a, 13b) gebildet ist.
